# EUROPÄISCHE PATENTSCHRIFT

(11) **EP 1 786 757 B1**
(45) Veröffentlichungstag und Bekanntmachung des Hinweises auf die Patenterteilung: **16.02.2011**
(21) Anmeldenummer: 05777554.6
(22) Anmeldetag: 16.08.2005
(51) Int. Cl.: C07C 69/712, C22B 3/00

(54) **SPEZIELLE CALIXARENE UND IHRE VERWENDUNG**
PARTICULAR CALIXARENES AND USE THEREOF
CALIXARENES PARTICULIERS ET LEUR UTILISATION

(30) Priorität: 24.08.2004 DE 102004040923
(43) Veröffentlichungstag der Anmeldung: 23.05.2007
(73) Patentinhaber: Bayer Technology Services GmbH, 51368 Leverkusen (DE); H.C. Starck GmbH, 38642 Goslar (DE)
(72) Erfinder: TRAVING, Michael, 51399 Burscheid (DE); GUTKNECHT, Wilfried, 38642 Goslar (DE); BÄCKER, Werner, 51688 Wipperfürth (DE); KUMMER, Wolfgang, 38642 Goslar (DE); LUDWIG, Rainer, 2444 Seibersdorf (AT)
(74) Vertreter: Lütjens, Henning
(86) Internationale Anmeldenummer: PCT/EP2005/008865
(87) Internationale Veröffentlichungsnummer: WO 2006/021342

(56) Entgegenhaltungen:
- EP-A- 0 237 265
- EP-A- 0 490 631
- GRADY, T. ET AL.: "Determination of the enantiomeric composition of chiral amines based on the quenching of the fluorescence of a chiral calixarene" ANAL. CHEM., Bd. 68, Nr. 21, 1996, Seiten 3775-3782, XP002364286
- DIAMOND, D. ET AL.: "Characteristics of sodium-selective electrodes based on a triethylester monoacid derivative of p-tert-Butylcalix[4]arene" ANALYTICAL PROCEEDINGS, Bd. 32, Nr. 4, 1995, Seiten 137-140, XP009060386
- BARRETT, G. ET AL.: "Selective monohydrolysis of bridged and unbridged calix[4]arene esters and its inhibition by alkali ion. Evidence for hydronium ion complexation" J. CHEM. SOC. PERKIN TRANS. 2, Bd. 9, 1992, Seiten 1595-1601, XP009060359 in der Anmeldung erwähnt
- LUDWIG, R. ET AL.: "New macrocyclic extractants for the processing of inorganic waste" NUKLEONIKA, Bd. 32, Nr. 2, 1998, Seiten 161-173, XP009060384 in der Anmeldung erwähnt
- CHAWLA, H.M. ET AL.: "Synthesis of 25,26,27-Tris(ethoxycarbonylmethoxy)-28-(4 -methyl-7-coumarinyloxycarbonylmethoxy)cal ix-4-arene" INDIAN JOURNAL OF CHEMISTRY, Bd. 32B, 1993, Seiten 1162-1164, XP009060385
- ARNAUD-NEU, F. ET AL.: "Selective alkali-metal cation complexation by chemically modified calixarenes. Part 4. Effect of substituent variation on the Na+/K+ selectivity in the ester series and X-ray crystal structure of the trifluoroethyl ester" J. CHEM. SOC. PERKIN TRANS. 2, Bd. 7, 1992, Seiten 1119-1125, XP009060397
- GROENEN, L.C. ET AL.: "syn-1,2-Dialkylated calix[4]arenes: General intermediates in the NaH/DMF tetraalkylation of calix[4]arenes" TETRAHEDRON LETTERS, Bd. 32, Nr. 23, 1991, Seiten 2675-2678, XP002364287
- IWAMOTO, K. ET AL.: "Remarkable metal template effects on selective syntheses of p-t-butylcalix[4]arene conformers" TETRAHEDRON LETTERS, Bd. 31, Nr. 49, 1990, Seiten 7169-7172, XP002364288 in der Anmeldung erwähnt
- IWAMOTO, K. ET AL.: "Conformations and structures of tetra-O-alkyl-p-tert-butylcalix[4]arenes. How is the conformation of calix[4]arenes immobilized?" J. ORG. CHEM., Bd. 56, 1991, Seiten 4955-4962, XP002364289 in der Anmeldung erwähnt
- INOKUCHI, F. ET AL.: "Selektive Erkennung von Alkalimetall-Kationen durch pi-basische, molekulare Hohlräume und einfacher massenspektrometrischer Nachweis von Kation-Aren-Komplexen" ANGEW. CHEM., Bd. 107, Nr. 12, 1995, Seiten 1459-1462, XP009060511 in der Anmeldung erwähnt

## Beschreibung

Die vorliegende Erfindung betrifft Calix[4]arene, welche Alkaliionen (Na⁺, K⁺) effektiv binden und über Phasengrenzen hinweg transportieren, und ihre Verwendung.

Die selektive Abtrennung von Alkaliionen kann die Produktqualität in der metallerzeugenden, der Polymer-, der biotechnologischen und anderen Industrien verbessern, indem eine höhere Reinheit und damit zusammenhängend neue bzw. bessere Materialeigenschaften beim Produkt erreicht werden. Bei solchen Trennungen liegen häufig saure bzw. basische Aufschlusslösungen vor, die in ihrem pH-Wert nicht beeinflusst werden sollen. Daher sollen nach Möglichkeit weder Säuren noch Basen (Ammoniak u.a.) mit abgetrennt werden.

Als Calix[4]arene werden Calix[n]arene bezeichnet, in denen n=4 Phenylringe miteinander verbrückt sind. (Gutsche (1998) Calixarenes Revisited, The Royal Society of Chemistry, Cambridge, UK; Ludwig (2000) Fresenius' J. Analyt. Chem., 367(2), 103-128) Infolge von Substitutionsreaktionen am molekularen Grundkörper sind zahlreiche Abkömmlinge bekannt. Einige dieser Derivate zeigen Selektivität gegenüber Metallionen, wie z. B. Pb(II), Au(III), Ln(III) bzw. Am(III). Diese Selektivität kann zu chemischen Trennungen genutzt werden. Diese Patentschrift beschreibt Calix[4]arene, deren Molekülstruktur so verändert wurde, dass selbst aus stark ammoniakalischen Lösungen die Alkaliionen Na⁺ und K⁺ selektiv mittels Flüssig-flüssig-Extraktion, Adsorption oder Membrantechniken entfernt werden können und dabei andere Metallionen vollständig in der Lösung verbleiben. Wir fanden überraschend, dass diese Aufgabe auch mit Calixarenen gelöst werden kann, die keine Kronenether-Gruppen tragen, wie dies in bisherigen Entwicklungen der Fall ist, die im folgenden zusammengefasst sind.

Die chemische Abtrennung von Alkaliionen Na⁺ und K⁺ zählt wegen ihrer kaum ausgeprägten Neigung zur Komplexbildung (Powell (2000) The ILTPAC Stability Constants Database, Academic Software) zu den schwierigsten Trennproblemen in der Chemie überhaupt. Der erste Lösungsansatz dazu gelang mit Kronenethern (Pedersen (1970) J. Amer. Chem. Soc., 92(2), 391-394; Strzelbicki & Bartsch (1981) Analyt. Chem., 53(12), 1894-1899; Hayashita, Goo, Lee, Kim, Krzykawski, & Bartsch (1990) Analyt. Chem., 62(21), 2283-2287; Bartsch, Hayashita, Lee, Kim, & Hankins (1993) Supramol. Chem., 1, 305-311; Bartsch & Hayashita (1999) ACS Symposium Series 716: Metal-Ion Separation and Preconcentration; Lindner, Toth, Jeney, Horvath, Pungor, Bitter, et al. (1990) Mikrochim. Acta [Wien], 1, 157-168; Imato, Honkawa, Kocsis, & Imasaka (1993) Proc. of the East Asia Conference on Chemical Sensors, 268-271; Bereczki, Agai, & Bitter (2003) J. Inclusion Phen. and Macrocyclic Chem., 47, 53-58; Izatt, Pawlak, Bradshaw, & Bruening (1991) Chem. Rev., 91(8), 1721-2085).

Kronenether sind jedoch für industrielle Anwendungen ungeeignet, da sie z. B. (i) bei Kontakt in die Haut eindringen was zu deren Absterben führt, (ii) etwas wasserlöslich sind und damit für einen kontinuierlichen Prozess wenig geeignet sind, und (iii) sehr teuer sind. Diese Verbindungsklasse hat deswegen nur im chemisch-analytischen und präparativen Bereich bis zu einem gewissen Grade Eingang gefunden.

Alle bekannten Techniken führen daher zu unbefriedigenden Ergebnissen bezüglich der selektiven Abtrennung von Alkaliionen bzw. zu damit verbundenen hohen Kosten.

Calixarene sind prinzipiell in der Lage, die drei genannten Nachteile zu vermeiden. Um einem Calixaren eine für praktische Anwendungen ausreichend hohe Selektivität für Na⁺ innerhalb der Gruppe der Alkaliionen zu verleihen, wurden bisher Kronenethergruppen in das Molekül integriert (Beer, Drew, Knubley, & Ogden (1995) J. Chem Soc. Dalton Trans. (19), 3117-3124; Koh, Araki, Shinkai, Asfari, & Vicens (1995) Tetr. Lett., 36(34), 6095-6098; Scheerder, Duynhoven, Engbersen, & Reinhoudt (1996) Angew Chemie, 108(10), 1172-1175; Shibutani, Yoshinaga, Yakabe, Shono, & Tanaka (1994) J. Inclusion Phen. and Molec. Recognition in Chem, 19, 333-342; Yamamoto, Sakaki, & Shinkai (1994) Chem Letters (3), 469-472; Yamamoto & Shinkai (1994) Chem Letters (6), 1115-1118; Yamamoto, Ueda, Suenaga, Sakaki, & Shinkai (1996) Chem. Letters, 39-40).

Einige dieser Molekülstrukturen wurden patentiert, wobei die Anwendung auf analytischem Gebiet liegt (Yamamoto & Ogata (1992) JP 04339251, 16 Nov.; Yamamoto & Shinkai (1994) JP 07206852, 8.8. 1995; Yamamoto (1995) JP Appl. H7-79787, 10.März; Yamamoto, Sakaki, & Shinkai (1995) JP 08291165, 5. Nov. 1996; Yamamoto & Shinkai (1996) JP 08245616, 24. 9.).

Die Kronenethergruppe hat folgende Funktionen im Molekül: (i) eine Versteifung der Struktur, so dass die funktionellen Gruppen in einer definierten, kaum veränderlichen Position im Raum gehalten werden, und (ii) einen Hohlraum bestimmter Größe zu formen. Wird die Kronenethergruppe oder eine ähnliche Gruppe wie z.B. eine Azakronenethergnippe verlängert, so wird der Ligand selektiv für K⁺ (Casnati, Pochini, Ungaro, Bocchi, Ugozzoli, Egberink, et al. (1996) Chem Europ. J., 2(4), 436-445; Kim, Shon, Ko, Cho, Yu, & Vicens (2000) J. Org. Chem, 65(8), 2386-2392; Shinkai (1994) JP6116261, 26.4.; Wenger, Asfari, & Vicens (1995) J. Inclusion Phen. and Molec. Recognition in Chem, 20, 293-296).

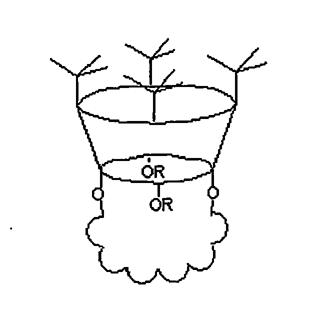

### (1) Schematische Darstellung eines verkrontes Calixarens, mit dem Calix[4)aren-Grundgerüst, eine Holraurnbildende Gruppe R=H oder weitere Substituenten und einem verbrückenden Kronether-Ring, wie z.B. -(OCH₂CH₂-)ₙ

Dieser strukturelle Ansatz mit Kronenethergruppen (1) lässt sich auch zur Herstellung Cs⁺-selektiver Calixarene anwenden (Dozol, Asfari, Hill, Vicens (1994) FR 2698362, 27. 5.; Dozol, Rouquette, Ungaro, & Casnati (1994), WO9424138, 20. 7. 1999; Moyer, Sachleben, Bonnesen, Presley (1999) WO 9912878, 18. 3.). Diese, auch als verkronte Calixarene bezeichnete Stoffgruppe ist nicht-toxisch und wasserunlöslich. Ihre Synthese ist jedoch aufwendig, da (i) die Kronenethergruppe mit einer definierten Länge in der schrittweisen Herstellung aufwendig und daher teuer ist, (ii) die Reaktionsausbeute wegen mehrerer möglicher Reaktionswege (Brückenbildung, Substitution ohne Brückenbildung) niedrig ist, und (iii) wegen der nicht-verbrückten Nebenprodukte eine aufwendige Reinigung mittels Chromatographie oder Phasentransfer erforderlich ist. Obwohl verkronte Calixarene die höchsten bekannten Selektivitäten für Alkaliionen aufweisen, fanden sie aus diesen Gründen bisher nur Eingang in analytische Verfahren bzw. in spezielle Trennungen von Radionukliden (Ludwig & Nguyen (2002) Sensors, 2, 397-416).

Calixarene ohne Kronenethergruppe wurden von Harris (Harris, McKervey, Svebla, & Diamond (1992) EP 0490631 A, US 5,132,345) zur analytischen Bestimmung der Alkaliionen Na⁺ bzw. K⁺ mittels ionensensitiver Elektroden vorgeschlagen. Wenngleich die Gruppe der offenbarten Calixaren-Derivate (2) in dieser Patentschrift sehr weit gefasst ist, so werden doch jene Verbindungen nicht eingeschlossen, bei denen ein Phenylring in die entgegengesetzte Richtung zeigt und die Carbonylgruppen gleichzeitig unterschiedliche Substituenten tragen, wie dies in (2) für eine cone-Konformation gezeigt ist. Die entsprechende Struktur (3) bezeichnet man als partielle *cone* Konformation.

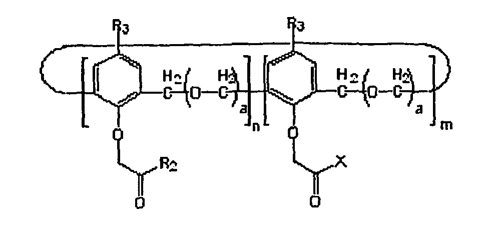

### (2) nach EP 0490631

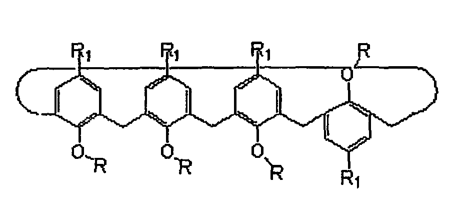

Partielle cone-Konformation in der Struktur (3), R und R1 können innerhalb des Moleküls variieren.

Calix[4]arene ohne kationenaustauschende Gruppe wurden ebenfalls von Harris et al. zu analytischen Zwecken vorgeschlagen (Cadogan, Diamond, Smyth, Deasy, McKervey, & Harris (1989) Analyst, 114(Dec.), 1551-1554). Eine Zusammenfassung zu ionophoren Eigenschaften der Calix[4]arene findet sich in einem mehrteiligen Werk (McKervey, Schwing-Weill, & Arnaud-Neu (1996) Molecular Recognition: Receptors for Cationic Guests. Comprehensive Supramolecular Chemistry, ed. G. W. Gokel, Pergamon Press, New York, Oxford, vol. 1, 537-603).

Die Einführung einer einzigen Carbonsäuregruppe in ein Calix[4]aren wurde erstmals von Böhmer et al. in einem Syntheseschritt realisiert (Barrett, Böhmer, Ferguson, Gallagher, Harris, Leonard, et al. (1992) J. Chem Soc., Perkin Trans. II, (9), 1595-1601; Böhmer, Vogt, Harris, Leonard, Collins, Deasy, et al. (1990) J. Chem Soc, Perkin Trans. I, (2), 431-432; Owens, McKervey, Böhmer, Vierengel, Tabatani, & Ferguson (1991) Workshop on Calixarenes and Related Compounds, Mainz 28.-30.8., p. 6). Dabei handelte es sich um *tert*-Butylcalixarene in *cone*-Konformation. Verbindungen dieser Struktur können als Ausgangsstoffe für weitere Derivate mit ionophoren Eigenschaften dienen (Ludwig, Tachimori, & Yamato (1998) Nukleonika, 43(2), 161-174).

Grady et al. (Grady, Cadogan, McKittrick, Harris, Diamond, & McKervey (1996) Analyt. Chimica Acta, 336, 1-12) berichten über die Anwendbarkeit des Monocarboxylates von *tert*-Butyl-calix[4]aren in ionensensitiven Elektroden für Na⁺. Auch hier handelt es sich um das *cone*Konformer. Im Vergleich zur Ester-Vorstufe wird keine Verbesserung in der Selektivität bezüglich Na⁺ beobachtet

Ebenfalls auf Na⁺ bezieht sich ein Verfahrenspatent von Reinhoudt (Reinhoudt, D. N., Engbersen, J. F. J., Peters, F. G. A. (2000) WO 2000029337, 25. 5.), in dem er ein Calix[4]aren mit 4 Estergruppen (Amaud-Neu, Collins, Deasy, Ferguson, Harris, Kaitner, et al. (1989) J. Amer. Chem. Soc., 111(23), 8681-8691; Chang & Cho (1986) J. Chem. Soc., Perkin Trans I, (2), 211-214; Kimura, Matsuo, & Shono (1988) Chem. Letters, (4), 615-616) bzw. mit einer COOH-Gruppe (Barrett, et al. (1992) J. Chem. Soc., Perkin Trans. II, (9), 1595-1601; Böhmer, et al. (1990) J. Chem. Soc., Perkin Trans. I, (2), 431-432) vorschlägt, die für ihre Na⁺-Selektivität bekannt sind, um aus Kreislaufwässern der holländischen Gewächshäuser das von Pflanzen nicht aufgenommene Na⁺ mittels Membranverfahren zu entfernen. Sein Verfahren zielt auf die Entfernung des für Pflanzen schädlichen Na⁺ mit Selektivität über K⁺ ab, wie die Verteilungsdaten beweisen.

Arnaud-Neu, F. et al untersuchten die selektive Alkalimetall-komplexierung von Tri- und Tetraesterderivaten in der cone-konformation (J. Chem. Soc. Perkin Trans. 2 (1992), S. (1119-1125).

Calix[4]arene können mit schwereren Metallionen, wie K⁺ und Cs⁺ eine zusätzliche Wechselwirkung eingehen, die auf der Beteiligung der aromatischen π-Systeme beruht und als Kationen-π-Wechselwirkung bezeichnet wird (Casnati (1997) Gazz. Chim. Ital., 127(11), 637-649; Inokuchi, Miyahara, Inazu, & Shinkai (1995) Angew. Chemie, 107(12), 1459-1461; Iwamoto, Araki, & Shinkai (1991) J. Org. Chem., 56(16), 4955-4962; Iwamoto, Fujimoto, Matsuda, & Shinkai (1990) Tetr. Lett., 31(49), 7169-7172). Dies wird bei Konformeren in alternierender oder in partieller *cone* Konformation beobachtet. Über eine Kombination mit einer -COOH-Gruppe bzw. eine Anwendung zu chemischen Trennungen bezüglich K⁺ wurde nicht berichtet.

Der Erfindung liegt die Aufgabe zugrunde, die Nachteile der bisher vorgeschlagenen Lösungen zu beseitigen und sowohl K⁺ als auch Na⁺ gemeinsam und mit hoher Selektivität, insbesondere über Ammoniumionen, zu binden und zu extrahieren, dies aber kostengünstig.

Die Aufgabe wurde überraschenderweise durch die Calix[4]arene in partieller *cone* Konformation gemäß der Ansprüche 1 und 2 gelöst. Die erfindungsgemäßen Calix[4]arene können Alkaliionen binden und besitzen eine Selektivität für Alkaliionen über Ammoniumionen.

Die Vorteile der erfindungsgemäßen Verbindungen liegen unter anderem darin, dass:
1) Bei der Herstellung durch die kurzen Reaktionszeiten sowie die einfache Aufarbeitung im Vergleich zu den verkronten Calixarenen Zeit gespart wird.
2) Bei der Herstellung und der Reinigung preiswerte Chemikalien eingesetzt werden können, wodurch sich die Kosten im Vergleich zu den verkronten Calixarenen, die Oligoethylenglycol-Ditosylate erfordern, deutlich verringern.
3) Mit der Verbindung entsprechend der Struktur (4) Nebenbestandteile wie K⁺ und Na⁺ von Hauptbestandteilen abgetrennt werden können. Im Gegensatz dazu beruhen konventionelle Verfahren der Extraktion bzw. Adsorption darauf, im Reinigungsschritt den Hauptbestandteil abzutrennen. Das neue Verfahren ist daher Ressourcen-schonend.

Die erfindungsgemäßen Calixarene besitzen die folgende Struktur (4): 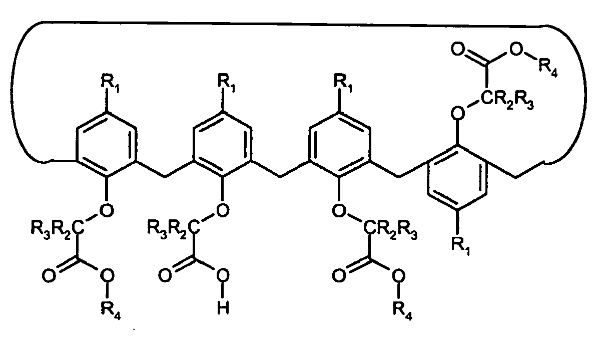 wobei
- R₁ =: H, Alkyl, Aryl, Arylalkyl, vorzugsweise verzweigte Alkylgruppen, z. B. *tert*-Butyl, *tert-* Octyl;
- R₂ =: H, F, Cl;
- R3 =: H, F, Cl, wobei R₂ gleich oder ungleich R₃ sein kann;
- R₄ =: substituierte oder unsubstituierte Alkyl-, Aryl-, Arylalkyl, Alkenylgruppe, vorzugsweise eine wasserabweisende, aber sterisch kleine Gruppe, z. B. Ethyl, Methyl, Propyl.

Bevorzugte Calixarene sind solche mit
- R₁ = *tert*-Butyl, R₂ = R₃ = H, R₄ = C₂H₅,
- R₁ = *tert*-Octyl, R₂ = R₃ = H, R₄ = C₂H₅,
- R₁ = H, R₂ = R₃ = H, R₄ = C₂H₅,
- R₁ = *tert*-Butyl, R₂ = R₃ = H, R₄ = CH₃,
   R₁ = *tert*-Octyl, R₂ = R₃ = H, R₄ = CH₃,
- R₁=H,R₂ = R₃ = H, R₄ = CH₃,
- R₁ = *tert*-Butyl, R₂ = R₃ = Cl, R₄ = C₂H₅,
- R₁ = *tert-*Octyl, R₂ = R₃ = Cl, R₄ = C₂H₅,
- R₁ =H, R₂=R₃=Cl, R₄=C₂H₅,
- R₁ = *tert*-Butyl, R₂ = H, R₃ = F, R₄ = C₂H₅,
- R₁ = *tert*-Octyl, R₂ = H, R₃ = F, R₄ = C₂H₅ oder
- R₁=H, R₂=H, R₃=F, R₄=C₂H₅,
wobei *tert*-Octyl = 1,1,3,3-Tetramethylbutyl ist.

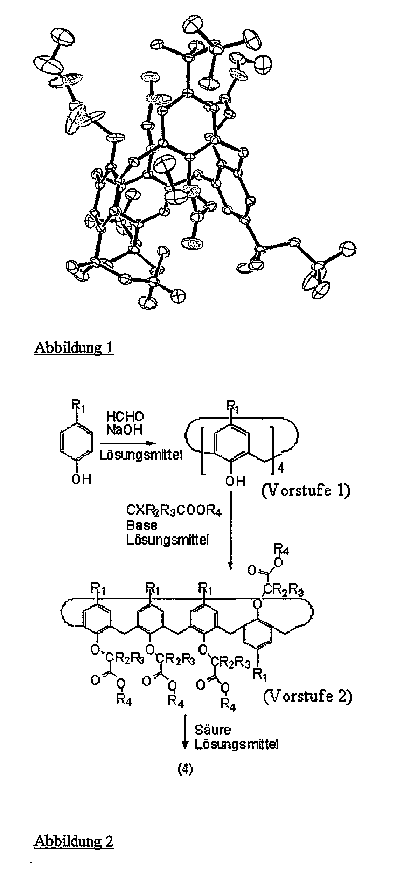

Abbildung 1 zeigt die mittels Röntgenstrukturanalyse bestimmte Struktur für R₁ = *tert*-Octyl (1,1,3,3-Tetramethylbutyl) und R₂ = R₃ = H. In Abbildung 1 sind die Kohlenstoffatome schwarz und die Sauerstoffatome grau gezeichnet, Wasserstoffatome sind nicht mit eingezeichnet.

Die erfindungsgemäßen Calixarene komplexieren K⁺ zugleich mit Na⁺, selbst bei gleichzeitiger Anwesenheit eines Überschusses an NH₄⁺/NH₃.

Diese Eigenschaft lässt sich mit folgenden Strukturmerkmalen erklären:
- Ein Calix[4]aren-Grundgerüst mit einem Hohlraumdurchmesser ähnlich dem der Alkaliionen Na⁺, K⁺.
- Genügend viele Sauerstoffatome, um Koordinationszahl und -geometrie der Alkaliionen abzusättigen.
- Eine partielle *cone* Konformation, die dem K⁺ mit seinen unbesetzten ³d-Orbitalen, nicht jedoch dem NH₄⁺ die Möglichkeit gibt, zusätzlich stabilisierende Kationen-π-Wechselwirkungen einzugehen und dadurch den Komplex zu stabilisieren.
- Eine einzige kationenaustauschende Gruppe, die eine Carbonsäure- oder eine alphahalogenierte Carbonsäuregruppe sein kann, und die Ladung des Alkaliions bei der Komplexierung kompensiert.
- Ein wasserabweisendes molekulares Gerüst, das der Verbindung folgende, für chemische Trennungen vorteilhafte Eigenschaften verleiht: (i) Wasserunlöslichkeit, (ii) Löslichkeit in organischen Verdünnungsmitteln bzw. gute Haftung auf porösen organischen Trägermaterialien, (iii) Gute Phasenkoaleszenz bei Extraktions- bzw. Membranverfahren.

Die erfindungsgemäßen Verbindungen sind kompatibel mit anderen Verbindungen wie z.B. kommerziellen Extraktionsmitteln oder anderen Calixarenen und können mit diesen zum Zwecke chemischer Trennungen kombiniert werden.

Die erfindungsgemäßen Verbindungen der Struktur (4) können überraschend einfach nach dem folgenden Verfahren erhalten werden:

Das Verfahren zur Herstellung der Calix[4]arene besteht darin, dass
A - Ein para-substituiertes Phenol, bevorzugt mit einer Verzweigung in der para-Gruppe, bevorzugt *tert*-Butylphenol oder *tert*-Octylphenol, mit Formaldehyd oder Paraformaldehyd und NaOH in katalytischen Mengen in einem hochsiedenden inerten Lösungsmittel, bevorzugt mit Siedebereich 200-260 °C und der Fähigkeit, mit dem Reaktionswasser ein Azeotrop zu bilden, z. B. Petrolether oder Diphenylether, zum Calix[4]aren (Abbildung 2) kondensiert wird. Diese Verbindungen, im weiteren als Vorstufe 1 bezeichnet, sind seit mehreren Jahrzehnten als Grundlagenwissen bekannt (Gutsche (1989) Calixarenes, (1998) Calixarenes Revisited, The Royal Society of Chemistry, Cambridge, UK, Schwetlick (2001) Organilam, Wiley-VCH).
B - Dieses Zwischenprodukt in Anwesenheit eines Überschusses einer Base, vorzugsweise einer starken Base mit p*K*_{B} < 3 und besonders bevorzugt einer starken Base mit Templateffekt wie KOAlkyl, z. B. KO*^{t}*Bu oder KO*ⁿ*Bu mit einfach oder mehrfach halogeniertem, bevorzugt in 1-Position chloriertem oder bromierten Essigsäurealkylester in einem inerten Lösungsmittel einer der folgenden Klassen, ohne sich auf diese Aufzählung zu beschränken, bei Temperaturen zwischen -10 und +150 °C, vorzugsweise zwischen 10 und 50 °C, innerhalb von einem Tag zur Reaktion gebracht wird. Als Lösungsmittel kommen dabei substituierte und unsubstituierte Ether, Alkane, Cycloalkane, Aromaten, Heterocyclen, Carbonsäureamide, Nitrile, vorzugsweise mit hohem Lösevermögen für die Reaktionspartner in Frage, wie z. B. Tetrahydrofuran. Das so erhaltene erste Derivat kann durch Waschen mit verdünnter Salzsäure von Resten an Salzen befreit und durch Umkristallisieren aus einem inerten Lösungsmittel, bevorzugt einem Alkohol, wie z. B. Ethanol, gereinigt werden.
C- Das so nach B hergestellte Derivat, Vorstufe 2, wird in einem inerten organischen Lösungsmittel, vorzugsweise mit hohem Lösevermögen für die Ausgangsstoffe, wie z.B. Tetrahydrofuran, Alkohol, Chloroform, mit einem großen Überschuss an Säure, vorzugsweise einer starken Säure, z. B. Salzsäure oder Trifluoressigsäure bei Temperaturen zwischen -10 und +150 °C, vorzugsweise 20 bis 40 °C, innerhalb von einem Tag zur Reaktion gebracht. Nach dem Waschen mit Wasser wird das reine Produkt mit der Struktur (4) erhalten. Das Produkt ist in der Lage, K⁺ und Na⁺ aus wässrigen Lösungen selektiv abzureichern. Wird die Anwendung zur Entfernung aus stärker sauren wässrigen Phasen verlangt, so sind R₂ oder R₃ bzw. R₂ und R₃ ein Halogen, vorzugsweise F oder Cl.

Die Synthese führt bei den hier beschriebenen Calix[4]arenen, vermutlich infolge von Templat-Effekten, überraschenderweise in hohen Reaktionsausbeuten zu den Derivaten mit der gewünschten Struktur.

Diese Templat-Effekte lassen sich, ohne den Gegenstand der vorliegenden Erfindung damit einzuschränken, wie folgt erklären:
- Der erste, überraschend gefundene TemplateSekt wird vermutlich durch die organischlösliche K⁺-haltige starke Base, wie z.B. KO*^{t}*Bu beim ersten Derivatisierungsschritt hervorgerufen und fixiert das Molekül in der gewünschten Konformation. Für R₁ = *tert-*Octyl ist dies die erste veröffentlichte Struktur in partieller *cone* Konformation. Ihre Herstellung unter den für R₁ *tert*-Butyl bisher bekannten Reaktionsbedingungen ist, wenn überhaupt, nur in Reaktionsausbeuten unter 2% der Theorie möglich, wie eigene Vorversuche ergaben.
- Der zweite, ebenso überraschende Templateffekt tritt auf, wenn das Produkt des ersten Syntheseschrittes mit einem ca. 20-fachen molaren Überschuss an Säure erhitzt wird. Wie die Kristallstruktur in Abb. 1 sowie die Kernresonanzdaten (vide infra) zeigen, wird nur die mittlere der drei benachbarten Estergruppen hydrolytisch gespalten. Im Gegensatz dazu wird eine partielle Hydrolyse des *cone*-Konformers bereits mit äquimolaren Mengen an Säure erreicht (Böhmer, et al. (1990) J. Chem. Soc., Perkin Trans I, (2), 431-432).

Die erfindungsgemäßen Verbindungen sind chemisch außerordentlich stabil, was sich darin äußert, dass sie ohne Einbuße an Trennwirkung mehrfach unter alkalischen (pH ≤ 11) bzw. sauren Bedingungen im Kreislauf Extraktion/Rückextraktion gefahren werden können, wie anhand der Kernresonanzspektren gezeigt werden konnte Die Verbindungen sind nicht-toxisch und vollständig unlöslich in der wässrigen Phase. Sie sind schwer entflammbare Feststoffe und unbegrenzt lagerbar.

Zur Anwendung wird die Verbindung mit der Struktur (4) allein oder im Gemisch mit weiteren Extraktions- oder Adsorptionsmitteln in einem chemisch inerten organischen Verdünnungsmittel, vorzugsweise mit niedrigem Dampfdruck, z. B. hochsiedenden Aliphaten, gelöst oder auf einem porösen Träger chemisch oder adsorptiv gebunden, vorzugsweise durch Belegung der Oberfläche makroporöser Strukturen, wie z. B. Polymeren.

In Kontakt mit wässriger Lösung, die außer K⁺ und Na⁺ auch einen großen Überschuss an Salzen anderer Metalle, an Ammonium, an anderen Basen oder an Säuren sowie an neutralen organischen Verbindungen enthalten kann, extrahiert die Verbindung mit hoher Selektivität die Alkaliionen K⁺ und Na⁺ und belässt die anderen Stoffe in der wässrigen Phase. Aus der beladenen organische Phase bzw. dem beladenen Adsorber werden die Alkaliionen durch Kontaktierung mit Säure, bevorzugt verdünnter Lösung einer starken Säure, wie z. B. 0.1 M Schwefelsäure, in wenigen Minuten, unabhängig von der Temperatur entfernt. Dabei wird das Extraktionsmittel in die ursprüngliche, unkomplexierte Form überführt und steht für einen neuen Zyklus zur Verfugung.

Die möglichen Anwendungen solcher Trennungen sind:
1) Entfernung von K⁺ und Na⁺ aus Prozesswässern, die einen Wertstoff enthalten. Der Wertstoff kann aus der gereinigten wässrigen Lösung gefällt werden und besitzt eine hohe Reinheit.
2) Entfernung von K⁺ und Na⁺ aus Kreislaufwässern in Produktionsprozessen, in denen sich K⁺ und Na⁺ anreichern. Wird der Komplexbildner auf einen festen Träger fixiert oder wird unter Verwendung der Komplexbildners ein Co-Polymerisat hergestellt, oder wird er als Feststoff direkt eingesetzt, bleiben die chemischen Eigenschaften erhalten und es findet eine Abtrennung von K⁺ und Na⁺ statt.
3) Gemeinsame Entfernung aller unerwünschten Verunreinigungen in wässrigen Lösungen einschließlich K⁺ und Na⁺, indem mindestens eine erfindungsgemäße Verbindung mit anderen Extraktions- oder Adsorptionsmitteln gemischt eingesetzt wird. Die Fähigkeit, K⁺ und Na⁺ zu binden, bleibt nämlich überraschenderweise auch im Gemisch mit anderen Verbindungen, die ihrerseits weitere Stoffe aus der wässrigen Phase entfernen, erhalten.
4) Die quantitative analytische Bestimmung des Gehaltes an K⁺ und Na⁺ in ammoniakalischen Lösungen, wenn mindestens eine erfindungsgemäße Verbindung in Verbindung mit einem Sensor verwendet wird. Die selektive Komplexierung ermöglicht die quantitative Gehaltsbestimmung der Alkaliionen K⁺ und Na⁺ in ammoniakalischen Lösungen, wenn eine erfindungsgemäße Verbindung als Teil eines Sensors verwendet wird. Ein Sensor kann z. B. eine ionensensitive Elektrode, ein modifizierter Feldeffekttransistor oder eine Optode sein.
5) Die Entfernung von Na⁺ und K⁺ aus wässrigen Lösungen und ihre Maskierung, so dass keine unerwünschten Nebenwirkungen mehr auftreten können. Die erfindungsgemäßen Verbindungen komplexieren und binden die K⁺ und Na⁺ selektiv gegenüber Ammoniumionen, anderen Kationen, Anionen und neutralen Verbindungen. Diese Selektivität kann zur Abtrennung mittels Flüssig-flüssig-Extraktion ausgenutzt werden.

Die Erfindung soll an einem Beispiel erläutert werden.

### Ausführungsbeispiel

### 1.) Herstellung

Zur Herstellung von (4) mit R₁ = *tert*-Octyl, R₂ = R₃ = H und R₄ = C₂H₅ werden folgende Synthesebedingungen angewandt:
- 10 Gramm *tert*-Octylcalix[4]aren (Cornforth, D'Arcy Hart, Nicholls, Rees, & Stock (1955) Brit. J. Pharmacol., 10, 73-86; Ohto, Yano, Inoue, Yamamoto, Goto, Nakashio, et al. (1995) Analyt. Sciences, 11, 893-902) werden mit 13 Gramm Kalium-*tert-*butanolat in 150 Milliliter getrocknetem Tetrahydrofuran am Rückfluss 4 Stunden lang erhitzt. Unter Rühren werden sodann ca. 9 Milliliter Bromessigsäureethylester zugetropft. Die Mischung wird ca. 1 Tag lang gerührt.
- Anstelle von THF können andere inerte Lösungsmittel, wie z.B. Petrolether verwendet werden. Reaktionsbeschleunigende Zusätze, wie z.B. KI können zu Beginn beigemischt werden. Wird zu Beginn außerdem K₂CO₃ (3 Gramm) zugegeben, so entsteht als Nebenprodukt ein cone-Isomer in etwa 10% Ausbeute, das mit dem Hauptprodukt zusammen auskristallisiert.
- Anstelle von Bromessigsäureethylester kann Chloressigsäureethylester verwendet werden, wobei anschließend auf ca. 50°C erwärmt wird.
- Das Reaktionsgemisch wird durch Zugabe von 50 Millilitern Wasser gequencht und mit 25 Millilitern CH₂Cl₂ extrahiert. Die organische Phase wird mehrmals mit ca. 0.5 molarer HCl gewaschen, eingeengt und durch Zugabe von ca. 25 Milliliter Ethanol zur Kristallisation gebracht.
- Der abfiltrierte und getrocknete Niederschlag (11 Gramm) wird in ca. 20 Millilitern CHCl₃ gelöst und mit 11 Millilitern Trifluoressigsäure 12 Stunden lang unter Rühren leicht erwärmt. Danach wird die organische Phase mehrmals mit Wasser bis zur neutralen Reaktion gewaschen. Das Lösungsmittel wird abgezogen und das Produkt verbleibt als harzartige, leicht gelbliche Masse, die beim Erwärmen erweicht.
- Anstelle von CHCl₃ kann auch ein anderes Lösungsmittel wie CH₂Cl₂, Tetrahydrofuran oder Alkohol verwendet werden. Anstelle von Trifluoressigsäure kann auch eine andere mittelstarke bis starke nichtoxydierende Säure verwendet werden. Die Säurekonzentration in der organischen Phase muss ausreichend hoch sein. Wird z. B. eine wässrige Lösung von HCl verwendet, muss deshalb mit einem wasserlösenden organischem Lösungsmittel wie Tetrahydrofuran gearbeitet werden.

### 2.) Identifizierung

Die Produkts wurden anhand ihrer NMR-Spektren, Kristallstrukturanalyse (vide supra), Massenspektren und Chromatogramme nachgewiesen:
Ester-Derivat mit R₁ = *tert*-Octyl, R₂ = R₃ = H, R₄ = C₂H₅: ¹H NMR, δ (ppm, CDCl₃/TMS) = 0.64 (s, 18H), 0.78 (s, 9H), 0.81 (s, 9H), 0.97 (s, 6H), 1.14 (t, 9H), 1.28 (t, 9H), 1.34 (t, 6H), 1.46 (m, 10H), 1.72 (s, 2H), 1.78 (s, 2H), 3.11 (d, 2H), 3.85 (s, 4H), 3.98 (q, 2H), 4.2 - 4.34 (m, 14H), 4.43 (d, 2H), 4.48 (d, 2H), 6.45 (s, 2H), 7.02 (s, 4H), 7.32 (s, 2H);
¹³C NMR, δ (ppm, CDCl₃/TMS) = C_{CH₃} und Ar-CH₂-Ar: 13.79, 14.04, 27.92, 30.41 - 32.59 (m), 36.67, 37.50, 37.66, 37.76, C_{CH₂}: 56.57, 57.38, 57.58, 59.22, 59.91, 60.28, 60.36, 67.59, 70.13, 71.19, C_{Ar}: 126.25, 126.64, 128.92, 130.88, 131.04, 131.65, 132.92, 134.73, 143.04, 143.18, 143.59, 152.13, 153.83, 154.83, C_{CO}: 168.87, 168.99, 170.84;
Elementaranalyse:, C₇₆H₁₁₂O₁₂, berechnet C 75.0%, H 9.3%, gefunden C 74.8%, H 9.06%.
mp. (Gallenkamp): 155 °C, DSC (Netzsch DSC 200 Calorimeter, N₂ , 10K/min) 149.7 °C (43.2 J/g);
MS (FAB⁺, 3kV, Xenon, Matrix MNBA) m/z = 1218 [L+H]⁺, 1240 [L+Na]⁺,
DC (SiO₂, CHCl₃/EtOH 9:1) R_{f}= 0.7 (zum Vergleich: *cone*Konformer R_{f}= 0,4, Ausgangsstoff R_{f} = 0.95);
Verbindung (4) mit R₁ = *tert*-Octyl, R₂ = R₃ = H, R₄ = C₂H₅: ¹H NMR, δ (ppm, CDCl₃/TMS) = 0.38 (s, 15H), 0.57 (s) und 0.62 (s) und 0.67 (s) (21H), 1.0 - 1.1 (m, 21H), 1.25 - 1.45 (m, 16H), 1.61 (s, 4H), 3.02 (d, 2H), 3.47 (s, 1H), 3.73 (d, 2H), 3.87 - 4.07 (m, 11H), 4.32 (s, 2H), 4.62 (d, 2H), 4.70 (d, 2H), 6.74 (s, 2H), 6.92 (s, 2H), 7.02 (s, 2H), 7.16 (s, 2H);
Elementaranalyse:, C₇₄H₁₀₈O₁₂, berechnet C 74.7%, H 9.15%, gefunden C 74.3%, H 8.85%.
mp.: erweicht ab 40 °C;
MS (FAB⁻, 3kV, Xenon, Matrix MNBA) m/z = 1188 [L-H]⁻; (FAB⁺)m/z = 1212 [L+Na]⁺;
DC (SiO₂, CHCl₃/EtOH 9:1) Rf = 0;

### 3.) Anwendung

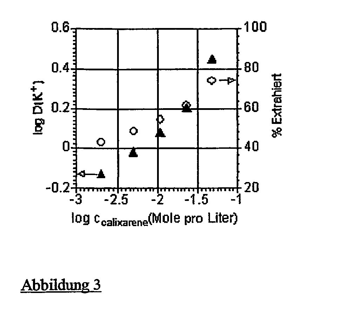

Mit (4) und R₁ = *tert*-Octyl, R₂ = R₃ = H, R₄ = C₂H₅ ergeben sich bei der Flüssig-flüssig-Extraktion die in Abb. 3 dargestellten Verteilungskoeffizienten *D* (*D* = Konzentration Metall in der org. Phase / Konz. Metall in der wässrigen Phase).

Abbildung 3: Logarithmus der Verteilungskoeffizienten von K⁺ bei der einstufigen Extraktion mit unterschiedlichen Konzentrationen an (4) mit R₁ = *tert*-Octyl, R₂ = R₃ = H, R₄ = C₂H₅ in CHCl₃ aus stark ammoniumhaltiger Lösung. Geschlossene Symbole: log D, offene Symbole: % extrahiertes K⁺.

Versuchsbedingungen: Lösung von Ligand (4) in Chloroform, 0.5 Mol/Liter NH₄Cl/NH₃-Puffer (pH 10), 200 ppm K⁺ vor der Extraktion, Analytik: FES.

Durch eine Erhöhung der Ligandkonzentration kann die Extraktionsausbeute in einfacher Weise gesteigert werden. Die Abreicherung an K⁺ in einer einstufigen Extraktion/Reextraktion um 50% und mehr erlaubt eine effektive Trennung.

## Patentansprüche

1. Calixarene in partieller cone-Konformation mit einer Struktur der Formel (4): 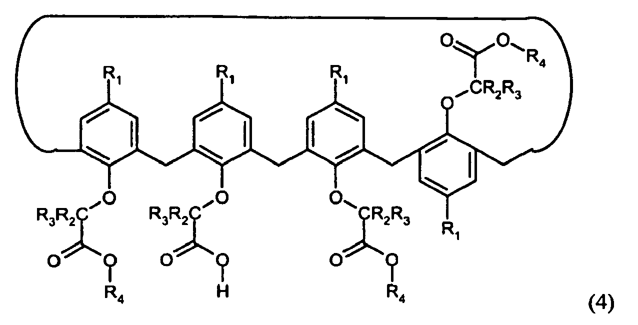 wobei
R₁ für H, Alkyl-, Aryl-, Arylalkylgruppen,
R2 für H, F, Cl,
R₃ für H, F, Cl und
R₄ für substituierte oder unsubstituierte Alkyl-, Alkenyl-, Aryl-, Arylalkylgruppen, stehen.

2. Calix[4]arene nach Anspruch 1 mit
- R₁ = *tert*-Butyl, R₂ = R₃ = H, R₄ = C₂H₅,
- R₁ = *tert*-Octyl, R₂ = R₃ = H, R₄ = C₂H₅,
- R₁=H, R₂=R₃=H, R₄=C₂H₅,
- R₁ = *tert*-Butyl, R₂ = R₃ = H, R₄ = CH₃,
- R₁ = *tert*-Octyl, R₂ = R₃ = H, R₄ = CH₃,
- R₁=H, R₂=R₃=H, R₄=CH₃,
- R₁ = *tert*-Butyl, R₂ = R₃ = Cl, R₄ = C₂H₅,
- R₁ = *tert*-Octyl, R₂ = R₃ = Cl, R₄ = C₂H₅,
- R₁=H, R₂ = R₃ = Cl, R₄ = C₂H₅,
- R₁ = *tert*-Butyl, R₂ = H, R₃ = F, R₄ = C₂H_{5,}
- R₁ = *tert*-Octyl, R₂ = H, R₃ = F, R₄ = C₂H₅ oder
- R₁ = H, R₂ = H, R₃ = F, R₄ = C₂H₅,
wobei tert-Octyl = 1,1,3,3-Tetramethylbutyl ist.

3. Verwendung von Calix[4]arenen nach Anspruch 1 oder 2, um die Alkaliionen K⁺ und Na⁺ zu binden.

4. Verwendung nach Anspruch 3 zur Extraktion der Alkaliionen K⁺ und Na⁺.

5. Verwendung nach Anspruch 3 zur Adsorption von K⁺ und Na⁺.

6. Verwendung von Calix[4]arenen nach Anspruch 3 im Gemisch mit anderen Extraktions- oder Adsorptionsmitteln.

7. Verwendung von Calix[4]arenen nach Anspruch 3 zur Bestimmung des Gehaltes an K⁺ und Na⁺.

## Claims

1. Calixarenes in partial cone conformation having a structure of the formula (4): 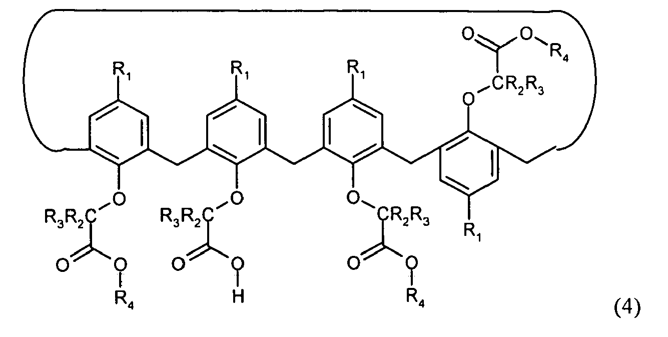 where
R₁ is H, or an alkyl, aryl or arylalkyl group,
R₂ is H, F, Cl,
R₃ is H, F, Cl and
R₄ is a substituted or unsubstituted alkyl, alkenyl, aryl or arylalkyl group.

2. Calix[4]arenes according to Claim 1 in which
- R₁ = *tert*-butyl, R₂ = R₃ = H, R₄ = C₂H₅,
- R₁ = *tert*-octyl, R₂ = R₃ = H, R₄ = C₂H₅,
- R₁ = H, R₂ = R₃ = H, R₄ = C₂H₅,
- R₁ = *tert*-butyl, R₂ = R₃ = H, R₄ = CH₃,
- R₁ = *tert*-octyl, R₂ = R₃ = H, R₄ = CH₃,
- R₁ = H, R₂ = R₃ = H, R₄ = CH₃,
- R₁ = *tert*-butyl, R₂ = R₃ = Cl, R₄ = C₂H₅,
- R₁ = *tert*-octyl, R₂ = R₃ = Cl, R₄ = C₂H_{5,}
- R₁ = H, R₂ = R₃ = Cl, R₄ = C₂H₅,
- R₁ = *tert*-butyl, R₂ = H, R₃ = F, R₄ = C₂H₅,
- R₁ = *tert*-octyl, R₂ = H, R₃ = F, R₄ = C₂H₅,
- R₁ = H, R₂ = H, R₃ = F, R₄ = C₂H₅,

3. Use of calix[4]arenes according to Claim i or 2 to bind the alkali metal ions K⁺ and Na⁺.

4. Use according to Claim 3 for the extraction of the alkali metal ions K⁺ and Na⁺.

5. Use according to Claim 3 for the adsorption of K⁺ and Na⁺.

6. Use of calix[4]arenes according to Claim 3 in admixture with other extractants or adsorption media.

7. Use of calix[4]arenes according to Claim 3 for determining the content of K⁺ and Na⁺.

## Revendications

1. Calixarènes en conformation conique partielle ayant une structure de formule (4) _{:} 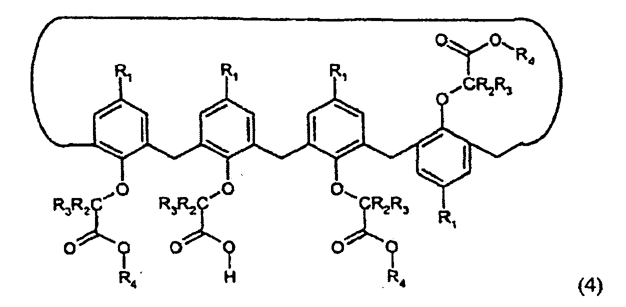 dans laquelle
R₁ représente H, des groupes alkyle, aryle, arylalkyle,
R₂ représente H, F, Cl,
R₃ représente H, F, Cl et
R₄ représente des groupes alkyle, alcényle, aryle, arylalkyle substitués ou non substitués.

2. Calix[4]arènes selon la revendication 1, avec
- R₁ = tert-butyle, R₂ = R₃ = H, R₄ = C₂H₅,
- R₁ = tert-octyle, R₂ = R₃ = H, R₄ = C₂H₅,
- R₁ = H, R₂ = R₃ = H, R₄ = C₂H₅,
- R₁ = tert-butyle, R₂ = R₃ = H, R₄ = CH₃,
- R₁ = tert-octyle, R₂ = R₃ = H, R₄ - CH₃,
- R₁ = H, R₂ = R₃ = H, R₄ = CH₃,
- R₁ = tert-butyle , R₂ = R₃ = Cl, R₄ = C₂H₅,
- R₁ = tert-octyle, R₂ = R₃ = Cl, R₄ = C₂H₅,
- R₁ = H, R₂ = R₃ = Cl, R₄ = C₂H₅,
- R₁ = tert-butyle, R₂ = H, R₃ = F, R₄ = C₂H₅,
- R₁ = tert-octyle, R₂ = H, R₃ = F, R₄ = C₂H₅ ou
- R₁ = H, R₂ = H, R₃ = F, R₄ = C₂H₅,
avec tert-octyle = 1,1,3,3-tétraméthylbutyle.

3. Utilisation de calix[4]arènes selon la revendication 1 ou 2, pour relier les ions alcalins K⁺ et Na⁺.

4. Utilisation selon la revendication 3 pour l'extraction des ions alcalins K⁺ et Na⁺.

5. Utilisation selon la revendication 3 pour l'adsorption de K⁺ et Na⁺.

6. Utilisation de calix[4]arènes selon la revendication 3 en mélange avec d'autres agents d'extraction ou d'adsorption.

7. Utilisation de calix[4]arènes selon la revendication 3 pour la détermination de la teneur en K⁺ et Na⁺ .
